# EUROPEAN PATENT SPECIFICATION

(11) **EP 2 084 255 B1**
(45) Date of publication and mention of the grant of the patent: **22.01.2014**
(21) Application number: 07824152.8
(22) Date of filing: 15.10.2007
(51) Int. Cl.: C11B 9/00

(54) **FRAGRANCE COMPOSITIONS**
GERUCHSSTOFFZUSAMMENSETZUNGEN
COMPOSITIONS DE PARFUM

(30) Priority: 23.10.2006 GB 0621020; 27.11.2006 US 604257
(43) Date of publication of application: 05.08.2009
(73) Proprietor: Givaudan Nederland Services B.V., 1411 GP Naarden (NL)
(72) Inventor: PERRING, Keith Douglas, Ashford Kent TN24 8HS (GB); BEHAN, John Martin, Ashford Kent TN25 4JB (GB); EVANS, Michael Gordon, Nonigton Kent CT15 4HH (GB); CHURCHILL, Anne, Canterbury Kent CT4 7LP (GB); CARE, Briony, Herne Bay Kent CT6 5H2 (GB)
(74) Representative: Simmons, John Murray
(86) International application number: PCT/GB2007/003901
(87) International publication number: WO 2008/050086

(56) References cited:
- EP-A- 0 955 035
- EP-A- 1 424 071
- EP-A- 1 661 973
- WO-A-02/36088

## Description

### Field of the Invention

The present invention relates to a method for providing a mood of well-being and happiness through the inhalation of a fragrance composition.

### Background

Well-being is a measure of happiness and self-satisfaction, and is assessed against personal base lines and expectations of what constitutes happiness and satisfaction. For some people, well-being means a need to feel joy, for others it is about contentment. There are several dimensions that lead to well-being. Among the most important of these are our physical, motivational and emotional needs.

Physical health is a major platform of well-being. Happy people have been shown to have a more robust immune status, and keeping happy may help firm up the body's defences and fight off disease.

Motivational needs refer to our life goals. There are three main dimensions here. First, for people to achieve well-being they must meet their basic needs such as food, partnership, spiritual beliefs etc. Next they should have achievable life goals. Finally the means of achieving these goals must be open. The person needs to believe that through their own endeavours, or with support, they can strive successfully. If any of these motivational dimensions is missing, the person is at risk of developing negative moods or even depression, with clear implications for their feelings of well-being.

The third dimension is emotional need. Although emotions fluctuate, when averaged over a longer period it has been shown that people have a mean level of emotion that exhibits a degree of stability over time. It is at this level that fragrance can play a role. If fragrance can cause change in a mood state, potentiate or reinforce a mood state, or make a mood state more agreeable then it can impact on emotional well-being and the overall state of the organism.

The literature disagrees as to how many emotions there are, however there is a consensus emerging that fear, joy, anger, disgust, distress and surprise are basic emotions experienced across all cultures. It has also been shown that these emotions are not learned; rather they are hard-wired into our brains (i.e. they involve non-verbal processing rather than reasoning). Photographs of facial expressions showing different emotions are understood by many cultures and societies as depicting the same experiences. Subjects experiencing happiness will display this fact by their behaviour, physiology, appearance or even their chemical aura. In this way they communicate their state of being to other subjects who are then able to share in the experience. Smiles can be infectious. Pleasant stimuli can act in the same way and their associated mood can be taken on by those who perceive them.

Emotions such as joy are transitory, brief events that rarely last more than a minute.

Happiness is a mood, and moods can last much longer, even hours. They are background states that provide a context for emotions. They can be both enablers and barriers.

Within a happy mood it is easier to experience joy. Conversely, within a sad mood it is difficult to feel joy. Moods input into our feelings of well-being, which in turn can be seen as the integration of emotional and physical inputs that come together to define our state.

Not surprisingly, the amount of time people experience pleasant versus unpleasant emotions is heavily weighted when reporting happiness. Frequent positive mood generation can have a disproportionate effect on overall well-being.

Humans are remarkably good at perceiving odours and have the capacity to discriminate an almost limitless number of different smells.

Some odours are universally pleasant such as sweet light floral notes, others universally unpleasant such as strong faecal and sulphurous notes. It may well turn out that this uniformity reflects a uniformity in our perception mechanisms and is as hard-wired as the emotions, "joy" and "fear". However, we can also learn to associate even extreme smells with more pleasant experiences. For example, consider the smell associated with some soft cheeses, where the short chain fatty acids that make these cheeses so palatable are seen as highly unpleasant when associated with smelly feet.

Fragrances are known to act at a rational level to reinforce positive associations, satisfy expectations, and build memorability of events and objects. They can also work on an emotional level to reinforce feelings.

We have discovered that, not unlike pictorial art, fragrances can provide a sensory stimulus from which, heretofore unknown, emotional benefits can flow and that these benefits can reinforce and promote well-being and happiness.

This invention relates to fragrance compositions that are targeted at delivering well-being benefits through frequent, low level positive mood stimulation. This and other objects are achieved by the delivery of fragrances for inhalation rather than through physical contact as found in aromatherapy.

The use of aromatic plants and oils dates back to ancient times. Holy perfumes were used as incense or aromatic oil to evoke a spiritual atmosphere or heighten spiritual awareness. In the times of the Assyrians and the Babylonians aromatic oils were popular in spas and baths. These people believed that they could use fragrant essences to preserve their health and thus live longer. Traditionally the art of aromatherapy is concerned with healing. It has been shown more recently that essential oils are absorbed through the mucosas of the mouth, nose, pharynx, gastro-intestines and also through intact skin. The oils interact with certain cell membrane lipids thus causing, among other effects, an alteration of the calcium-ion-channel-function. Besides this direct molecular action there is also stimulation in the limbic system in the brain which is responsible for all our emotions and sensations such as anxiety, fear, feelings of wellness, harmony and sexual desires. Through this mode of action aromatherapy has several therapeutic benefits, for beauty, general well-being, emotional help and certain illnesses. Treatments may be used as an effective method of stress relief or they may help people to become more energized.

Our research has shown that the ability of odours to enhance mood is related to their complexity. Complex odours in a similar olfactory category were generally more effective than simple blends, and simple blends were more effective than single materials and oils. In experiments, which involved pure smelling, as opposed to skin application and massage, the more complex creations outperformed their simple analogues in terms of mood generation. In all cases, perfumes developed according to the skills of the art, outperformed the simpler aromatherapy oils (see Example section). Apart from the widespread usage of aromatherapy there are few disclosures in the literature that reveal how to design complex fragrances to deliver target emotional benefits. European patent number EP 1,343,466 describes perfumes that will aid relaxation and calming, and PCT, patent application WO 2006/136828 describes how to formulate enlivening perfumes using a relatively limited perfume ingredient palette. US patent Number 7,097,863 discloses a process for relaxing the back, shoulder or neck muscles through the inhalation of aroma notes described as rosy, floral, musky, ambery, sweet and/or powdery. EP 1,218,023 relates to fragranced personal care compositions that may be used to calm mammals, including humans and in particular, humans aged between about 1 day to 12 years. These compositions include sensory components comprising a specified essential oil together with named perfume ingredients. EP 955035 describes body odour-preventing compositions containg fragrance compositions.

EP 1424971 describes psycho-sedative and psycho-stimulative perfume compositions.

We have discovered a route to creating 'happy' fragrances that cover a wide spectrum of hedonics and odour types yet deliver the desired emotional benefits upon inhalation. It is based on rules of composition that lead to a perfume formulation range which has a high probability of being perceived as 'happy'. Fragrances need to be perceived in an appropriate context. As stated earlier it is very difficult to experience joy if one is unhappy. Fragrance can reinforce happiness if a subject is in a condition to allow happiness to manifest itself. It has been found that a range of fruity/floral fragrances as developed according to the guidelines provided can deliver a happy effect, as reported by naive subjects (in a state allowing perception of enhanced happiness).

### Summary of the Invention

According to the present invention there is provided a method that provides a mood of well-being and happiness through the inhalation of an effective amount of a fragrance composition comprised of at least 75% by weight, preferably 85 % by weight of perfume materials drawn from the following groups:
A) At least 10% by weight in total of at least three materials drawn from Group 'HMP' comprising: 1-(2,6,6,8-tetramethyltricyclo [5.3.1.0{1,5}]undec-8-en-9-yl)ethanone; allyl cyclohexylpropionate; allyl heptanoate; Apple Oliffac S pcmf; 7-methyl-2H-1,5-benzodioxepin-3(4H)-one; cassis base; cis-3-hexenyl salicylate; damascenone; gamma-decalactone; ethyl acetoacetate; ethyl maltol; ethyl methyl phenylglycidate; hexyl acetate; (3E)-4-methyldec-3-en-5-ol; 2,5,5-trimethyl-6,6-bis(methyloxy)hex-2-ene; 4-(4-hydroxyphenyl)butan-2-one; styrallyl acetate; 2,2,5-trimethyl-5-pentylcyclopentanone; ylang oil.
B) Optionally up to 95% of materials from the following groups:
   Group 'HMR'
      comprising: allyl ionone; benzyl acetate; cis-jasmone; citronellol; ethyl linalol; ethylene brassylate; 4-methyl-2-(2-methylpropyl)tetrahydro-2H-pyran-4-ol; geraniol; geranium oil; iso-eugenol; lemon oil; 3-(4-hydroxy-4-methylpentyl) cyclohex-3-ene-1-carbaldehyde; 4-(4-hydroxy-4-methylpentyl)cyclohex-3-ene-1-carbaldehyde; alpha-iso-methyl ionone; 3-methylcyclopentadec-2-en-1-one; cyclopentadecanone; cyclohexadecanolide; gamma-undecalactone.
   Group 'HMI'
      comprising: 1-{[2-(1,1-dimethylethyl)cyclohexyl]oxy}butan-2-ol; 3a,6,6,9a-tetramethyldodecahydronaphtho[2,1-{b}]furan; alpha-damascone; dihydromyrcenol; eugenol; 3-(1,3-benzodioxol-5-yl)-2-methylpropanal; 2,4-dimethylcyclohex-3-ene-1-carbaldehyde; mandarin oil; orange oil; 2-(1,1-dimethylethyl)cyclohexyl acetate.
   Group 'RMP'
      comprising: anisic aldehyde; (2Z)-2-ethyl-4-(2,2,3-trimethylcyclopent-3-en-1-yl)but-2-en-1-ol; benzoin siam resinoid; ethyl vanillin; oxacyclohexadec-12(13)-en-2-one; hexyl salicylate; hydroxycitronellal; jasmin oil; 3-methyl-5-phenylpentan-1-ol; 2-(phenyloxy)ethyl 2-methylpropanoate; alpha-terpineol; vanillin;
   Group 'IMP'
      comprising: allyl amyl glycolate; benzyl salicylate; bergamot oil; coriander oil; cyclamen aldehyde; 1-(2,6,10-trimethylcyclododeca-2,5,9-trien-1-yl)ethanone; prop-2-enyl (cyclohexyloxy)acetate; Damascenia 185 SAE; 2,4-dimethylheptan-1-ol; fir balsam; fir needle oil; 3-(4-ethylphenyl)-2,2-dimethylpropanal; ginger oil; guaiacwood; linalyl acetate; litsea cubeba oil; methyl 2,4-dihydroxy-3,6-dimethylbenzoate; nutmeg oil; olibanum oil; orange flower oil; Ozonal AB 7203C; patchouli oil; rose oxide; rosemary oil; sage clary oil; spearmint oil; Tamarine AB 8212E; tarragon oil;
   Group 'GEN'
      comprising: cyclopentadecanolide; hexyl cinnamic aldehyde; ionone beta; isobornyl cyclohexanol; 1-(2,3,8,8-tetramethyl-1,2,3,4,5,6,7(8),8(8a)-octahydronaphthalen-2-yl)ethanone; 3-[4-(1,1-dimethylethyl)phenyl]-2-methylpropanal; linalol; methyl dihydrojasmonate; 2-phenylethanol; provided the following conditions are met:
      (a) HMPs + HMRs > = IMPs
      (b) HMPs + HMIs > = RMPs
      (c) HMRs + HMIs + HMPs + GENs > = 65%
      (d) HMPs/(HMPs+RMPs+IMPs) > = 0.35
      (e) HMPs/[(HMPs+RMPs+IMPs) + (100-TOTAL)] > = 0.2
wherein 'HMPs' indicates the sum of the percentages of materials within Group HMP, and similarly for the remaining groups, the symbol '>=' indicates 'at least equal to', and 'TOTAL' is the sum of HMPs, HMRs, HMIs, IMPs, RMPs and GENs, provided also that low odour or no odour solvents are excluded from the calculation of these sums. References herein the percentage by weight of perfume ingredients means relative to the total weight of perfume ingredients in the perfume composition and excludes, for example, any optional solvents, diluents, etc.

Disclosed herein are perfume formulation rules that those skilled in the art of perfumery can use to create perfumes with a high probability of promoting positive mood states associated with feelings of well-being and happiness.

We have carried out extensive studies into the relationship between perfume formulations and the mood states engendered upon inhalation of the perfumes. A variety of techniques have been used such as brain activity mapping, visual mood profiling and biophysical monitoring. Typical methodologies and output are described below.

The way in which a complex mixture of perfume ingredients affect mood is not well understood. Perfume materials that are associated with a mood state such as relaxing may nevertheless be useful in fragrance formulations that deliver the opposite effect. This ambiguity varies from material to material, and must be taken into account when creating fragrances designed for specific moods.

Without being bound by theory, on the basis of our observations we have classified perfume ingredients as belonging to various mood classes. This has enabled a number of predictive perfume creation rules to be constructed that are very useful in practice. The rules are based on recognising that membership of a class may be 'fuzzy', for example certain materials may be useful as building materials for a 'happy' fragrance but equally well may be useful in other perfume formulations to support say, relaxing moods or invigorating moods. Certain materials have been found to be useable in any fragrance (at the levels specified herein) to support a variety of mood states.

The classes are identified as follows: 'HMP' comprising perfume ingredients strongly associated with happy moods; 'HMR' comprising ingredients that may support both happy and relaxing moods; 'HMI' comprising ingredients that may support both happy and invigorating moods; 'RMP' comprising ingredients that strongly support relaxing moods; 'IMP' comprising ingredients that strongly support invigorating moods; and 'GEN; comprising ingredients that may support a variety of moods. It must be emphasised that these designations are relevant to ingredients as used by one skilled in the art (e.g. a perfumer) under the dosage and pattern constraints disclosed here.

### Materials

The below list details the materials of the invention, giving the common name of each material as used within the perfume industry, alongside with (where possible or where relevant) the corresponding IUPAC name and/or tradename(s) and suppliers. Certain materials (e.g. mandarin oil) are complex mixtures whose exact compositions may vary with geography and season. In such cases here the invention is taken to refer to the general class of oils irrespective of origin.

### Group HMP:

1-(2,6,6,8-tetramethyltricyclo [5.3.1.0{1,5}] undec-8-en-9-yl) ethanone, available under the names: Acetyl Cedrene, Vertofix Coeur (TM) (IFF), and Methyl Cedryl Ketone; allyl cyclohexyl propionate also known as prop-2-enyl 3-cyclohexylpropanoate; allyl heptanoate also known as prop-2-enyl heptanoate; Apple Oliffac S pcmf (TM) (IFF); 7-methyl-2H-1,5-benzodioxepin-3(4H)-one available under the name Calone 1951 (TM)(CAL); Cassis base; cis-3-hexenyl salicylate also known as (3Z)-hex-3-enyl 2-hydroxybenzoate; damascenone also known as (2E)-1-(2,6,6-trimethylcyclohexa-1,3-dien-1-yl)but-2-en-1-one; gamma-decalactone also known as decalactone gamma and 5-hexyldihydrofuran-2(3H)-one; ethyl acetoacetate also known as ethyl 3-oxobutanoate; ethyl maltol also known as 2-ethyl-3-hydroxy-4H-pyran-4-one; ethyl methyl phenylglycidate also known as ethyl 3-methyl-3-phenyloxirane-2-carboxylate; hexyl acetate; (3E)-4-methyldec-3-en-5-ol available under the names Jadenol (Q) and Undecavertol (G); 2,5,5-trimethyl-6,6-bis(methyloxy)hex-2-ene available under the name Methyl Pamplemousse (G); 4-(4-hydroxyphenyl)butan-2-one also known as Raspberry Ketone or Rastone; styrallyl acetate also known as 1-phenylethyl acetate; 2,2,5-trimethyl-5-pentylcyclopentanone available under the name Veloutone (F); ylang oil also known as ylang ylang oil.

### Group HMR:

Allyl ionone also known as (1E)-1-(2,6,6-trimethylcyclohex-2-en-1-yl)hepta-1,6-dien-3-one;
benzyl acetate also known as phenylmethyl acetate;
cis-jasmone also known as 3-methyl-2-[(2Z)-pent-2-enyl]cyclopent-2-en-1-one;
citronellol also known as 3,7-dimethyloct-6-en-1-ol;
ethyl linalol also known as (6Z)-3,7-dimethylnona-1,6-dien-3-ol;
ethylene brassylate also known as 1,4-dioxacycloheptadecane-5,17-dione;
4-methyl-2-(2-methylpropyl)tetrahydro-2H-pyran-4-ol available under the names Florosa (TM) (G) or Florol (TM) (F);
geraniol also known as (2E)-3,7-dimethylocta-2,6-dien-1-ol;
geranium oil;
iso eugenol also known as 2-(methyloxy)-4-[(1E)-prop-1-enyl]phenol;
lemon oil;
3-(4-hydroxy-4-methylpentyl)cyclohex-3-ene-1-carbaldehyde and 4-(4-hydroxy-4-methylpentyl)cyclohex-3-ene-1-carbaldehyde and mixtures thereof (eg as available under the name Lyral (TM) (IFF));
alpha-iso-methyl ionone also known as methyl ionone alpha iso and (3E)-3-methyl-4-(2,6,6-trimethylcyclohex-2-en-1-yl)but-3-en-2-one;
3-methylcyclopentadec-2-en-1-one available under the name Muscenone (TM) (F);
cyclohexadecanolide and cyclopentadecanone and mixtures thereof, e.g. as available under the name Silvanone (TM) (G);
gamma-undecalactone also known as undecalactone gamma, 5-heptyldihydrofuran-2(3H)-one, and Peche Pure (TM) (G).

### Group HMI:

1-{[2-(1,1-dimethylethyl)cyclohexyl]oxy}butan-2-ol available under the name Amber Core (TM) (G);
3a,6,6,9a-tetramethyldodecahydronaphtho[2,1-{b}]furan available under the names Amberlyn Super (TM) (G) and Cetalox (TM) (F);
alpha-damascone also known as damascone alpha and (2E)-1-(2,6,6-trimethylcyclohex-2-en-1-yl)but-2-en-1-one;
dihydromyrcenol also known as 2,6-dimethyloct-7-en-2-ol;
eugenol also known as 2-(methyloxy)-4-prop-2-enylphenol;
3-(1,3-benzodioxol-5-yl)-2-methylpropanal available under the names Aquanal™ (G) and Helional (TM) (IFF);
2,4-dimethylcyclonex-3-ene-1-carbaldehyde also know as Ligustral™(G);
mandarin oil;
orange oil;
2-(1,1-dimethylethyl)cyclohexyl acetate available under the names Ortholate (TM) (G) and Verdox (TM) (IFF).

### Group RMP:

anisic aldehyde also known as 4-(methyloxy)benzaldehyde;
(2Z)-2-ethyl-4-(2,2,3-trimethylcyclopent-3-en-1-yl)but-2-en-1-ol available under the names Bangalol (TM) (G) and Bacdanol (TM) (IFF);
Benzoin Siam resinoid;
ethyl vanillin also known as 3-(ethyloxy)-4-hydroxybenzaldehyde;
oxacyclohexadec-12(13)-en-2-one available under the name Habanolide (TM) (F);
hexyl salicylate also known as hexyl 2-hydroxybenzoate;
hydroxycitronellal also known as 7-hydroxy-3,7-dimethyloctanal;
jasmin oil;
3-methyl-5-phenylpentan-1-ol also known as Mefrosol (TM) (G);
2-(phenyloxy)ethyl 2-methylpropanoate also known as phenoxyethyl isobutyrate and available as a major component in Prunella (TM) (F);
alpha-terpineol also known as terpineol or 2-(4-methylcyclohex-3-en-1-yl)propan-2-ol; vanillin also known as 4-hydroxy-3-(methyloxy)benzaldehyde.

### Group IMP:

allyl amyl glycolate also known as prop-2-enyl [(2-methylbutyl)oxy]acetate;
benzyl salicylate also known as phenylmethyl 2-hydroxybenzoate;
bergamot oil;
coriander oil;
cyclamen aldehyde also known as 2-methyl-3-[4-(1-methylethyl)phenyl]propanal;
1-(2,6,10-trimethylcyclododeca-2,5,9-trien-1-yl)ethanone available under the name Cyclisone (TM) (G);
prop-2-enyl(cyclohexyloxy)acetate also know as allyl (cyclonhexyloxy)acetate and cyclogalbanate;
Damascenia 185 SAE (TM) (F);
2,4-dimethylheptan-1-ol also known as dimethyl heptanol;
fir balsam;
fir needle oil;
3-(4-ethylphenyl)-2,2-dimethylpropanal available under the name Floralozone (TM) (IFF);
ginger oil;
guaiacwood;
linalyl acetate also known as 1-ethenyl-1,5-dimethylhex-4-enyl acetate;
litsea cubeba oil;
methyl 2,4-dihydroxy-3,6-dimethylbenzoate also known as Moss Oakmoss Synthetic; nutmeg oil;
olibanum oil also know as olibanum resinoid;
orange flower oil;
Ozonal AB 7203C (TM)(G);
patchouli oil;
rose oxide also known as 4-methyl-2-(2-methylprop-1-enyl)tetrahydro-2H-pyran (including racemic and chiral forms);
rosemary oil;
sage clary oil;
spearmint oil;
tamarine AB 8212E (TM);
tarragon oil.

### Group GEN:

cyclopentadecanolide also known as oxacyclohexadecan-2-one;
hexyl cinnamic aldehyde also known as (2E)-2-hexyl-3-phenylprop-2-enal;
ionone beta also know as beta-ionone and (3E)-4-(2,6,6-trimethylcyclohex-1-en-1-yl)but-3-en-2-one;
isobornyl cyclohexanol also known as 3-(5,5,6-trimethylbicyclo[2.2.1]hept-2-yl)cyclohexanol and available under the name Sandela (TM) (G);
1-(2,3,8,8-tetramethyl-1,2,3,4,5,6,7(8),8(8a)-octahydronaphthalen-2-yl)ethanone available under the name Iso E Super (TM) and Isoambois (TM);
3-[4-(1,1-dimethylethyl)phenyl]-2-methylpropanal available under the name lily aldehyde;
linalol also known as 3,7-dimethylocta-1,6-dien-3-ol;
methyl dihydrojasmonate also known as methyl (3-oxo-2-pentylcyclopentyl)acetate;
2-phenylethanol also know as phenyl ethyl alcohol and phenylethanol.

Key to suppliers:
F = Firmenich
G = Givaudan
IFF = International Flavors and Fragrances
CAL = Calone

### Preferences

In preferred fragrance compositions, the sum of the percentages of ingredients falling within Groups HMR, HMI, HMP and GEN is at least 75%, and the ratio HMPs/ (HMPs + RMPs + IMPs) is at least 0.55, preferably at least 0.75. Also preferred are fragrance compositions that comprise at least five ingredients that are members of Group HMP, even more preferably at least eight ingredients.

Desirably the fragrance compositions also comprise at least one material from Group HMR, one from Group HMI, or one from each group. Additionally, the fragrance composition may comprise at least 5% by weight, or even at least 10 % by weight, in total of materials drawn from the total of Groups HMR and HMI.

Generally perfumes of the invention will contain at least 15 perfumery ingredients (including those not classified herein, but excluding solvents), and it is preferred that they contain at least 25 ingredients, even more preferred 40 ingredients. For the purposes of calculating numbers of ingredients, materials present at less than 0.1% may be ignored, and essential oils are counted as single materials.

Most preferred are perfumes comprising at least 40 perfume ingredients, 10% by weight of ingredients drawn from the Group HMP, and furthermore, the sum of the percentages of ingredients falling within Groups HMR, HMI, HMP and GEN is at least 75 % and the ratio HMPs/(HMPs+RMPs+IMPs) is at least 0.75.

The fragrance compositions of the invention exert a mood benefit via olfactory sampling of an effective amount of the perfume through inhalation. The inhalation may be of the headspace above the perfume itself, or above consumer products including the perfume, for example alcoholic systems such as colognes, bath and body preparations, air fresheners, and many others. As used herein "enlivening" refers to the psychological aspects of well-being, namely the feeling of happiness. Examples of desired responses of happiness range from simple contentment to intense joy. The term "effective amount" refers to the percentage by weight of the enlivening fragrance that is needed to be incorporated into a consumer product to promote the desired state of well-being and happiness.

The fragrance compositions may be used "as is" (e.g., 100%) or in a "cologne". Directions for quantity to use and frequency of use, as well as variations in the formulation, e.g., summer and winter formulations, may be employed to assure that effective levels of enlivening fragrance(s) may be administered. For the purpose of this invention, the term "cologne", as exemplified hereinafter, means an enlivening fragrance incorporated in an alcoholic or hydroalcoholic solution. The enlivening fragrance can vary between 1 to 99 % and the balance of the formulation is comprised of alcohol or a mixture of water and alcohol. The water:alcohol weight ratio can vary from 50:50 to 0:100. Examples of alcohols typically used in these products are SDA 39-C and SDA-40, either 190 "proof" or anhydrous (See "Ethyl Alcohol Handbook", 5th Edition, Published by National Distillers and Chemical Co.). The cologne can also contain solubilizing agents, emollients, humectants, thickening agents, bacteriostats or other cosmetically used ingredients.

### Solvents

A number of liquids are commonly used within perfumery as solvents for difficult to dissolve, intractable compounds such as solids or gums, or as carriers for powerful materials with intense odours. Examples of such solvents include dipropylene glycol (DPG), diethyl phthalate (DEP), benzyl benzoate, isopropyl myristate (IPM), triacetin and triethyl citrate (TEC). Typically these materials have no or very low odour and make little contribution to the overall odour of a perfume when present at normal concentrations (less than 60 %, usually less than 40 %, often less than 20%). For the purposes of this invention, such vehicles are not included in the calculations of the percentage compositions, so that, for example a perfume mixture containing 50% w/w of DPG together with 10% each of five perfume materials would be considered to be a perfume with the five materials at 20 % w/w each. This solvent correction is necessary to provide a true picture of the odour-bearing fraction of a perfume.

'No or very low odour' means that is scores less than 80 on an odour index scale as set out in EP0404470 (based on a comparison with the odour intensity of a control sample of a 10 % solution of benzyl acetate in dipropylene glycol, which corresponds to an index of 100), and includes diethylphthelate, dipropylene glycol, triacetin, benzyl benzoate, triethyl citrate, Herculyn D (trade mark), isopropyl myristate and acetyl tributylcitrate.

### Consumer Compositions

As used herein, "consumer composition" includes, but is not limited to, room fresheners or room deodorants; clothes deodorants; fabric softeners; dryer-added fabric softener articles; household cleansers; toilet bowl cleaners; cosmetic products such as powders, creams, deodorants, hand lotions and sun screens; personal care products such as antiperspirant and underarm deodorants, general body deodorants, hair care products such as hair sprays, conditioners, rinses, shampoos; foot care products; colognes, after shaves and body lotions; solid or liquid soaps and anionic, cationic, nonionic or zwitterionic detergents; odour control products; perfumed polymers; space odorants; colognes, toilet waters, hair preparations, such as lacquers, brilliantines, and pomades.

Consumer compositions may take a variety of forms including, but not limited to, powders such as talc, dusting powders, face powders and the like, bars, sticks, tablets, mousses, gels, liquids, sprays, fabric conditioning sheets, cleansing compositions, powders, oils, bath oils and other bath compositions, aerosols, candles, substances that may be used with vaporisers, wipes, washes, shampoos, gels, soaps, sticks, balms, sachets, pillows, mousses, sprays, lotions, creams and cleansing compositions.

When fragrance compositions are used as an olfactory component of a consumer composition, such as a solid or liquid anionic, cationic, nonionic or zwitterionic detergent or a cosmetic powder or a deodorant stick, as little as 0.1 % by weight of the fragrance composition in the perfumed article will suffice. In space odorant applications, on the other hand, as much as 99 % of the combined carrier perfume substance and fragrance composition(s) can be present. Thus, consumer compositions may contain in the range of from about 0.1 % up to about 99 % of a composition of matter consisting essentially of fragrance composition(s) of this invention.

The term "consumer composition" also includes solid-form polymers, such as polyethylene, polypropylene and other polymers that contain pores. Such perfumed polymers can be produced according to any technique well known to one having ordinary skill in the art.

### Dispensing

Dispensing of the fragrance compositions may be by any conventional means, such as from a vessel containing the odorant substance, optionally with a valve and nozzle mechanism, an aerosol or non-aerosol spray, a gas, a solid or liquid air freshener, a scented cloth, lotion, cream, perfume, cologne, potpourri, incense, light bulb ring, a candle, fabric softener, carpet shampoo or freshener, a plug-in air freshener, and the like. The fragrance compositions can be dispensed in combination with an odourless liquid carrier such as mineral oil or water, and can be formulated with a viscosity effective to allow for aerosolisation.

The fragrance compositions may also be packaged as a part of an article of manufacture, or kit. The kit can include in association, for example, (a) a carrier and other optional additives for forming a composition, placed in containing means such as a vial, jar, pouch, can, bottle, cloth, aerosol can, blister pack, and the like, containing an effective amount of an enlivening fragrance; and (b) means for instructing as to the enlivening fragrance and its use to promote well-being and happiness. The parts of the kit can be contained or separately packaged within a packaging material, such as a box or bag.

The fragrance compositions can be delivered in the form of a liquid solution, aerosol spray, solid, microcapsules, or other suitable form to deliver a suprathreshold amount of the odorant for sniffing and inhalation into the nasal passageway. The fragrance compositions can be administered in combination with an odourless liquid carrier such as mineral oil or water, and can be formulated with a viscosity effective to allow for aerosolisation. The enlivening fragrances can be dispensed, for example, by means of a cloth material that is coated with enlivening fragrances, as a solid or liquid form contained in a capped vessel, from an aerosol or pump-type spray device, as a nasal spray, by opening a blister pack or scratch-and-sniff odour patch containing the odorant in the form of microspheres, from a pen-like dispenser containing a liquid form of the fragrance compositions adsorbed to a wicking material, and the like.

Delivery of the fragrance compositions may employ a device that is portable and minimally disruptive of bystanders. The fragrance compositions can also be administered to a group of people within a confined area, for example, by pumping air containing enlivening fragrance through an air vent, spraying the enlivening fragrances into the air as a mist or dry powder using an aerosol or non-aerosol spray, and the like.

### Examples

The invention will be further described by reference to the following detailed examples, wherein the methodologies are as described below. These examples are not meant to limit the scope of the invention that has been set forth in the foregoing description. Variations within the concepts of the invention are apparent to those skilled in the art. Exposure to fragrances of this invention promoted positive affect or moods and led to enhanced ability to make associations between ideas and concepts, and to see more different similarities (or differences) among stimuli. The stimuli could be consumer products, and specifically could be characterised as mildly pleasant.

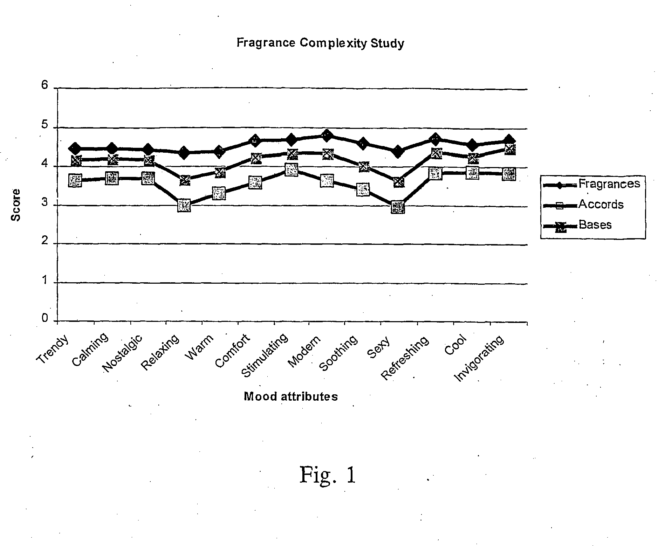

Figure 1 is a graph of smelling score against a variety of mood attributes for fragrance compositions described as being either a base, an accord or a finished fragrance.

### Example 1: A Study on Perfume Complexity

A total of 20 different odours were evaluated in a consumer sniff test. The odours were grouped according to their complexity into 3 groups: complex finished fragrances, mixes of fragrance materials known as accords, and simple fragrance mixes or bases. The consumers were asked to smell a subset of the odours and score against the attributes: trendy, calming, nostalgic, relaxing, warm, comforting, stimulating, modern, soothing, sexy, refreshing, cool energising. The results shown in figure 1 indicate that the more complex fragrances outperformed their simpler analogues across each of these attributes.

### Example 2: Mood Attribute Testing

This was a central location sniff test carried out in the UK. 150 naive consumers were either pre-recruited or recruited by intercept in the street to attend a hall test. They were pre-screened for any nasal disorders or allergic sensitivities to smelling fragrance. The sample of people used were all female and they were selected to represent a cross section of ages from 18 to 55 and a cross section of social classes.

Each subject was asked to smell 10 fragrances in a predetermined order. As each fragrance was smelt they were asked to mark a series of given mood scales according to how the fragrance made them feel (e.g. comforted, safe, soothed). There were 24 mood scales in all and each was scored on a 0 - 10 scale from "not at all" to "extremely". The ratings for 'happy' are shown for 8 perfumes in Table 1 below. Scores above 4.5 were taken as indicative of mood activation.

**Table 1: Mood Attribute Data**

| Code | Mean Scores 'happy' |
|---|---|
| H4 | 5.669 |
| H3 | 4.911 |
| H2 | 4.713 |
| H1 | 4.52 |
| C4 | 4.5 |
| C3 | 4.373 |
| C2 | 4.09 |
| C1 | 3.777 |

Table 2 and 4 below detail the fragrance compositions of Table 1 in w/w% terms, wherein fragrances entitled 'H1', 'H3' and 'H4' are fragrances according to the invention and those named 'C1', 'C2', 'C3', 'C4' and 'H2' are comparative examples. Table 3 and 5 detail the analysis of these compositions in terms of the Groups and conditions described herein. Note that '0.0' is correct to two significant places.

**Table 2: Fragrance Compositions**

| **INGREDIENT** | **GROUP** | | | | |
|---|---|---|---|---|---|
| | | **C1** **w/w%** | **C2** **w/w%** | **C3** **w/w%** | **H1** **w/w%** |
| ALLYL AMYL GLYCOLATE (G) | IMP | 0.0 | 0.0 | 0.0 | 0.2 |
| AMBERLYN SUPER (TM) (G) 10 % DPG | HMI | 2.0 | 0.0 | 0.0 | 0.0 |
| BANGALOL (TM)(G) | RMP | 0.0 | 0.0 | 3.0 | 0.0 |
| BANGALOL LAEVO (TM)(G) | RMP | 0.0 | 1.0 | 0.0 | 0.5 |
| BENZOIN SIAM RESINOID 50% DPG | RMP | 0.0 | 0.0 | 4.0 | 0.0 |
| BENZYL ACETATE | HMR | 0.0 | 3.0 | 0.0 | 0.5 |
| BERGAMOT OIL | IMP | 18.0 | 5.0 | 3.0 | 0.0 |
| CALONE 1951(TM) | HMP | 0.0 | 0.0 | 0.0 | 0.1 |
| CASSIS BASE | HMP | 0.0 | 0.0 | 0.0 | 1.8 |
| CIS 3 HEXENOL | NA | 0.0 | 0.0 | 0.0 | 0.1 |
| CIS 3 HEXENOL 10% DPG | NA | 0.0 | 0.0 | 2.0 | 0.0 |
| CIS 3 HEXENYL SALICYLATE | HMP | 3.0 | 5.0 | 0.0 | 2.0 |
| CIS JASMONE | HMR | 0.0 | 0.0 | 0.0 | 0.3 |
| CIS-6-NONEN-1-OL 0.1% DPG | NA | 0.0 | 0.0 | 0.0 | 0.5 |
| CITRONELLOL | HMR | 0.0 | 0.0 | 0.0 | 1.0 |
| COUMARIN | NA | 0.0 | 0.0 | 1.0 | 0.0 |
| CYCLAMEN ALDEHYDE | IMP | 0.0 | 0.0 | 0.0 | 1.0 |
| CYCLISONE (TM) | IMP | 0.0 | 3.0 | 0.0 | 0.0 |
| CYCLOGALBANATE 10% DPG | IMP | 0.0 | 0.0 | 0.0 | 0.8 |
| DAMASCENONE 10% DPG | HMP | 0.0 | 0.0 | 0.0 | 0.5 |
| DECALACTONE GAMMA | HMP | 0.0 | 0.0 | 0.0 | 0.3 |
| DIHYDROMYRCENOL | HMI | 0.0 | 0.0 | 3.0 | 1.0 |
| DIPROPYLENE GLYCOL (DPG) | SOLV | 0.0 | 9.8 | 0.0 | 11.4 |
| ETHYL ACETOACETATE | HMP | 0.0 | 0.0 | 0.0 | 0.5 |
| ETHYLENE BRASSYLATE | HMR | 8.0 | 0.0 | 16.0 | 0.0 |
| GALAXOLIDE (TM) (IFF) | NA | 0.0 | 0.0 | 0.0 | 10.0 |
| GERANIOL | HMR | 0.0 | 0.0 | 0.0 | 3.0 |
| HELIONAL (TM) (IFF) | HMI | 3.0 | 2.0 | 0.0 | 3.0 |
| HELIOTROPIN | NA | 0.0 | 0.0 | 1.0 | 0.0 |
| HEXYL CINNAMIC ALDEHYDE | GEN | 0.0 | 3.0 | 0.0 | 0.0 |
| HYDROXYCITRONELLAL | RMP | 0.0 | 0.0 | 0.0 | 2.5 |
| INDOLE 1% DPG | NA | 0.0 | 0.0 | 0.0 | 0.5 |
| IONOL (preservative) | NA | 0.0 | 0.0 | 0.0 | 0.2 |
| IONONE BETA 10% DPG | GEN | 0.0 | 0.0 | 0.0 | 0.4 |
| ISOAMBOIS (G) | GEN | 4.0 | 7.0 | 0.0 | 0.0 |
| ISOEUGENOL | HMR | 0.0 | 0.0 | 0.0 | 0.1 |
| JADENOL (TM) (G) | HMP | 0.0 | 0.0 | 0.0 | 4.0 |
| JASMOLACTONE (F) | NA | 0.0 | 0.0 | 0.0 | 0.1 |
| LAVENDER OIL | NA | 0.0 | 0.0 | 18.0 | 0.0 |
| LIGUSTRAL (G) | HMI | 0.0 | 0.1 | 0.0 | 0.2 |
| LIGUSTRAL (G) 10% DPG | HMI | 4.0 | 0.0 | 0.0 | 0.0 |
| LILY ALDEHYDE | GEN | 6.0 | 5.0 | 0.0 | 20.0 |
| LIME OIL | NA | 0.0 | 0.0 | 0.0 | 0.4 |
| LINALOL | GEN | 0.0 | 3.0 | 0.0 | 5.5 |
| LITSEA CUBEBA OIL | IMP | 1.0 | 0.4 | 0.0 | 0.0 |
| LYRAL | HMR | 0.0 | 0.0 | 5.0 | 0.0 |
| MANDARIN ITALIAN OIL | HMI | 4.0 | 3.0 | 0.0 | 0.0 |
| MANDARIN ITALIAN OIL 10% DPG | HMI | 4.0 | 0.0 | 0.0 | 0.0 |
| MANZANATE (G) 10% DPG | NA | 0.0 | 0.0 | 0.0 | 0.1 |
| MELONAL (TM) (G) | NA | 0.0 | 0.0 | 0.0 | 0.2 |
| METHYL ANTHRANILATE | NA | 0.0 | 0.4 | 0.0 | 0.0 |
| METHYL BUTANOL 1,2 10% DPG | NA | 0.0 | 0.0 | 0.0 | 0.2 |
| METHYL CHAVICOL 10% DPG | NA | 0.0 | 0.0 | 0.0 | 0.5 |
| METHYL DIHYDROJASMONATE | GEN | 36.0 | 42.0 | 10.0 | 15.0 |
| METHYL HEPTENONE 10% DPG | NA | 0.0 | 0.0 | 0.0 | 0.3 |
| METHYL HEPTINE CARBONATE 10% DPG | NA | 0.0 | 0.0 | 0.0 | 0.4 |
| METHYL IONONE ALPHA ISO | HMR | 0.0 | 0.0 | 12.0 | 4.0 |
| MOSS OAKMOSS SYNTHETIC 10% DPG | IMP | 2.0 | 0.0 | 3.0 | 0.2 |
| OLIBANUM RESINOID | IMP | 2.0 | 0.2 | 3.0 | 0.0 |
| ORTHOLATE (TM) (G) | HMI | 0.0 | 0.0 | 0.0 | 0.5 |
| OXANE (TM) (F) 1% DPG | U | 0.0 | 0.0 | 0.0 | 0.5 |
| OZONAL AB 7203C (TM) | IMP | 0.0 | 0.4 | 0.0 | 0.0 |
| PASSION FRUIT 109223 (F) | U | 0.0 | 0.7 | 0.0 | 0.0 |
| PATCHOULI LIGHT OIL | IMP | 0.0 | 0.0 | 3.0 | 0.0 |
| PHENYL ETHYL ALCOHOL | GEN | 0.0 | 5.0 | 0.0 | 1.0 |
| PRUNELLA (TM) (F) | RMP | 0.0 | 0.0 | 6.0 | 0.0 |
| SILVANONE (TM) (G) | HMR | 0.0 | 0.0 | 4.0 | 0.0 |
| TONALID | U | 0.0 | 0.0 | 0.0 | 4.5 |
| TOP ROSE BASE | U | 0.0 | 0.0 | 0.0 | 0.2 |
| TRANS-2-CIS-6-NONADIENAL 0.1% DPG | U | 0.0 | 1.0 | 0.0 | 0.0 |
| VANILLIN | RMP | 0.0 | 0.0 | 3.0 | 0.0 |
| | total % | | 100.0 | 100.0 | 100.0 |

| | | | | | |
|---|---|---|---|---|---|
| KEY B = BIOLANDES F = FIRMENICH G = GIVAUDAN IFF = INTERNATIONAL FLAVOR & FRAGRANCES Notes: 1) "Ingredient Y X% DPG" indicates X% of ingredient Y in a dipropylene glycol solution 2) "NA" indicates a material that is not allocated (i.e. the group is unknown). 3) "SOLV" indicates the material to be a solvent | | | | | |

**Table 3**

| GROUP ANALYSIS of Fragrance Compositions of Table 1 | | | | |
|---|---|---|---|---|
| **Group Sums** % | **C1** | **C2** | **C3** | **H1** |
| HMRs | 8.0 | 3.0 | 37.0 | 8.9 |
| HMIs | 11.0 | 5.1 | 3.0 | 4.5 |
| GENs | 46.0 | 65.0 | 10.0 | 41.5 |
| IMPs | 21.2 | 9.0 | 9.3 | 1.2 |
| HMPs | 3.0 | 5.0 | 0.0 | 8.8 |
| RMPs | 0.0 | 1.0 | 14.0 | 3.0 |
| TOTAL | 89.2 | 88.1 | 73.3 | 67.9 |
| SOLV | 10.8 | 10.8 | 6.5 | 15.9 |
| | | | | |

| **Solvent Corrected Sums** | **C1** | **C2** | **C3** | **H1** |
|---|---|---|---|---|
| HMRs | 9.0 | 3.4 | 39.6 | 10.6 |
| HMIs | 12.3 | 5.7 | 3.2 | 5.4 |
| GENs | 51.6 | 72.9 | 10.7 | 49.4 |
| IMPs | 23.8 | 10.1 | 9.9 | 1.5 |
| HMPs | 3.4 | 5.6 | 0.0 | 10.4 |
| RMPs | 0.0 | 1.1 | 15.0 | 3.6 |
| GENs + HMPs+HMIs+HMRs | 76.2 | 87.6 | 53.5 | 76.1 |
| TOTAL | 100.0 | 98.8 | 80.5 | 80.8 |

| **RATIOS** | | | | |
|---|---|---|---|---|
| HMPs/(HMPs+IMPs+RMPs) | 0.12 | 0.33 | 0.00 | 0.67 |
| HMPs/(HMPs+IMPs+RMPs+M) | 0.12 | 0.31 | 0.00 | 0.30 |

| | | | | |
|---|---|---|---|---|
| Key: TOTAL = HMPs+IMPs+RMPs+HMIs+HMRs+GENs M = 100 - TOTAL | | | | |

**Table 4: Fragrance Compositions**

| **INGREDIENT** | **GROUP** | **H2** w/w% | **H3** w/w% | **H4** w/w% | **C4** w/w% |
|---|---|---|---|---|---|
| ALLYL AMYL GLYCOLATE (G) | IMP | 0.0 | 0.2 | 0.0 | 0.6 |
| ALLYL CYCLOHEXYLPROPIONATE | HMP | 0.0 | 0.0 | 0.2 | 0.0 |
| ALLYL HEPTANOATE | HMP | 0.0 | 0.0 | 0.1 | 0.0 |
| AMBER CORE (TM) (G) | HMI | 0.0 | 1.5 | 0.0 | 0.6 |
| AMBERLYN SUPER (TM) (G) | HMI | 0.0 | 0.1 | 0.2 | 0.0 |
| AMBERLYN SUPER (TM) (G) 10% DPG | HMI | 1.0 | 0.0 | 0.0 | 2.1 |
| AMBRETTOLIDE (TM) (G) | NA | 0.0 | 0.0 | 0.3 | 0.0 |
| AMYL SALICYLATE | NA | 1.0 | 0.0 | 0.0 | 0.0 |
| APPLE OLIFFAC S PCMF (TM)(IFF) | HMP | 0.4 | 0.0 | 0.0 | 0.0 |
| ARMOISE TUNISIAN OIL | NA | 0.0 | 0.0 | 0.0 | 0.5 |
| BANGALOL (TM)(G) | RMP | 0.5 | 0.4 | 0.0 | 0.5 |
| BASIL COMORES OIL 10% DPG | NA | 0.0 | 0.0 | 0.0 | 0.3 |
| BENZALDEHYDE 10% DPG | NA | 0.1 | 0.0 | 0.0 | 0.0 |
| BENZYL ACETATE | HMR | 4.0 | 0.4 | 0.7 | 0.0 |
| BENZYL SALICYLATE | IMP | 0.0 | 0.0 | 0.0 | 7.0 |
| BERGAMOT OIL | IMP | 4.0 | 0.0 | 0.0 | 15.0 |
| BIRCH LEAF | NA | 0.4 | 0.0 | 0.0 | 0.0 |
| CALONE 1951(TM) | HMP | 0.0 | 0.1 | 0.0 | 0.0 |
| CALONE 1951(TM) 1% DPG | HMP | 0.0 | 0.0 | 0.6 | 0.0 |
| CAMOMILE MOROCCAN OIL | NA | 0.0 | 0.0 | 0.0 | 0.2 |
| CASSIS BASE | HMP | 0.3 | 1.8 | 2.6 | 0.0 |
| CASSIS BASE 10% DPG | HMP | 0.0 | 0.0 | 0.0 | 2.0 |
| CEDRENYL ACETATE | NA | 0.0 | 0.0 | 0.0 | 1.3 |
| CETALOX (TM) (F) | HMI | 0.0 | 0.1 | 0.0 | 0.0 |
| CIS 3 HEXENOL | NA | 0.0 | 0.1 | 0.1 | 0.0 |
| CIS 3 HEXENOL 10% DPG | NA | 1.0 | 0.0 | 0.0 | 0.0 |
| CIS 3 HEXENYL ACETATE 10% DPG | NA | 1.0 | 0.0 | 0.0 | 0.0 |
| CIS 3 HEXENYL METHYL CARBONATE 10% DPG | NA | 0.0 | 0.0 | 0.0 | 0.0 |
| CIS 3 HEXENYL SALICYLATE | HMP | 2.5 | 2.0 | 2.5 | 0.0 |
| CIS JASMONE | HMR | 0.1 | 0.2 | 0.1 | 0.0 |
| CIS-6-NONEN-1-OL 0.1% DPG | NA | 0.0 | 0.5 | 0.0 | 0.0 |
| CITRONELLOL | HMR | 0.0 | 0.8 | 1.0 | 0.0 |
| CITRONELLYL ACETATE | NA | 0.0 | 0.0 | 0.5 | 0.0 |
| CORIANDER OIL | IMP | 0.0 | 0.0 | 0.0 | 0.6 |
| COUMARIN | NA | 0.0 | 0.0 | 0.0 | 0.7 |
| CUMIN SEED 10% DPG | NA | 0.0 | 0.0 | 0.0 | 0.7 |
| CYCLAMEN ALDEHYDE | IMP | 0.0 | 0.1 | 0.0 | 1.0 |
| CYCLISONE (TM) | IMP | 0.0 | 0.0 | 0.0 | 1.0 |
| CYCLOPENTADECANOLIDE | GEN | 1.5 | 2.0 | 1.2 | 0.0 |
| CYCLOGALBANATE 10% DPG | IMP | 0.0 | 0.8 | 0.0 | 0.0 |
| DAMASCENIA 185 SAE (F) (TM) | IMP | 0.1 | 0.0 | 0.0 | 0.0 |
| DAMASCENONE 10% DPG | HMP | 0.0 | 0.3 | 0.5 | 0.0 |
| DAMASCONE ALPHA 10% DPG | HMI | 0.8 | 0.0 | 0.8 | 0.3 |
| DIHYDROMYRCENOL | HMI | 1.0 | 0.9 | 0.0 | 6.5 |
| DIMETHYLHEPTANOL | IMP | 0.0 | 0.0 | 0.0 | 1.8 |
| DIPROPYLENE GLYCOL (DPG) | SOLV | 3.9 | 0.0 | 14.1 | 3.5 |
| ETHYL ACETOACETATE | HMP | 0.0 | 0.2 | 0.4 | 0.0 |
| ETHYL BUTYRATE 10% DPG | NA | 0.0 | 0.0 | 0.1 | 0.0 |
| ETHYL LINALOL | HMR | 0.0 | 0.0 | 3.6 | 0.0 |
| ETHYL MALTOL | HMP | 0.0 | 0.0 | 0.3 | 0.0 |
| ETHYLENE BRASSYLATE | HMR | 3.0 | 11.9 | 12.3 | 0.0 |
| EUGENOL | HMI | 0.2 | 0.0 | 0.0 | 0.1 |
| FIR BALSAM OIL | IMP | 0.0 | 0.0 | 0.0 | 0.4 |
| FIR NEEDLE SIBERIA OIL | IMP | 0.0 | 0.0 | 0.0 | 2.0 |
| FLORALOZONE (IFF) | IMP | 0.0 | 0.0 | 0.0 | 2.0 |
| FLORALOZONE (IFF) 10% DPG | IMP | 0.2 | 0.0 | 0.0 | 0.0 |
| FLORHYDRAL (G) | NA | 0.0 | 0.3 | 0.0 | 0.0 |
| FLOROSA (TM) (G) | HMR | 0.0 | 3.0 | 2.4 | 0.0 |
| GALBANUM ARTESSENCE (B) 1% DPG | NA | 0.5 | 0.0 | 0.0 | 0.0 |
| GERANIOL | HMR | 0.0 | 3.0 | 0.5 | 0.0 |
| GERANIUM AFRICAN OIL | HMR | 0.1 | 0.0 | 0.0 | 0.0 |
| GERANYL ACETATE | NA | 0.0 | 0.0 | 0.0 | 0.3 |
| GINGER PURE | IMP | 0.0 | 0.0 | 0.0 | 0.4 |
| HABANOLIDE (TM) (F) | RMP | 2.0 | 0.0 | 0.0 | 0.0 |
| HELIONAL (TM) (IFF) | HMI | 3.0 | 5.8 | 1.2 | 3.0 |
| HEXYL ACETATE | HMP | 0.0 | 0.0 | 0.8 | 0.0 |
| HEXYL CINNAMIC ALDEHYDE | GEN | 5.0 | 0.0 | 0.0 | 0.0 |
| HYDROXYCITRONELLAL | RMP | 0.0 | 2.5 | 0.0 | 0.0 |
| INDOLE 1% DPG | NA | 0.0 | 0.5 | 0.0 | 0.0 |
| IONOL (preservative) | NA | 0.2 | 0.0 | 0.2 | 0.0 |
| IONONE ALPHA | NA | 0.0 | 0.0 | 0.2 | 0.0 |
| IONONE BETA | GEN | 0.8 | 0.0 | 1.1 | 0.0 |
| IONONE BETA 10% DPG | GEN | 0.0 | 0.4 | 0.0 | 0.0 |
| ISOAMBOIS (G) | GEN | 0.0 | 2.0 | 7.0 | 5.0 |
| ISOAMYL ACETATE 10% DPG | NA | 0.2 | 0.0 | 0.0 | 0.0 |
| ISOEUGENOL | HMR | 0.0 | 0.1 | 0.0 | 0.0 |
| ISOEUGENOL 10% DPG | HMR | 0.5 | 0.0 | 0.0 | 0.0 |
| JASMIN OIL | RMP | 0.2 | 0.0 | 0.0 | 0.0 |
| JASMOLACTONE (F) | NA | 0.0 | 0.1 | 0.0 | 0.0 |
| LAVANDIN ABRIALIS OIL | NA | 0.0 | 0.0 | 0.0 | 1.5 |
| LEMON ITALIAN OIL | HMR | 5.0 | 0.0 | 0.2 | 0.0 |
| LIGUSTRAL (G) | HMI | 0.0 | 0.2 | 0.0 | 0.0 |
| LIGUSTRAL (G) 10% DPG | HMI | 2.0 | 0.0 | 0.0 | 3.0 |
| LILY ALDEHYDE | GEN | 14.0 | 9.5 | 5.0 | 0.0 |
| LIME OIL | NA | 0.0 | 0.2 | 0.0 | 0.0 |
| LINALOL | GEN | 1.0 | 5.2 | 0.0 | 2.6 |
| LINALYL ACETATE | IMP | 0.0 | 0.0 | 0.0 | 8.5 |
| LYRAL | HMR | 2.0 | 10.0 | 0.0 | 1.3 |
| MANDARIN ITALIAN OIL | HMI | 0.4 | 0.0 | 1.3 | 0.0 |
| MANZANATE (G) 10% DPG | NA | 0.0 | 0.0 | 0.2 | 0.0 |
| MELONAL (TM) (G) | NA | 0.2 | 0.2 | 0.0 | 0.0 |
| METHYL CHAVICOL 10% DPG | NA | 0.0 | 0.5 | 0.0 | 0.0 |
| METHYL DIHYDROJASMONATE | GEN | 20.0 | 17.0 | 25.5 | 0.0 |
| METHYL HEPTENONE 10% DPG | NA | 0.0 | 0.3 | 0.0 | 0.0 |
| METHYL HEPTINE CARBONATE 10% DPG | NA | 0.0 | 0.2 | 0.0 | 0.0 |
| METHYL IONONE ALPHA ISO | HMR | 1.0 | 4.0 | 0.0 | 0.0 |
| METHYL OCTINE CARBONATE 20 10% DPG | NA | 0.7 | 0.0 | 0.0 | 0.0 |
| METHYL PAMPLEMOUSSE (G) | HMP | 0.0 | 0.0 | 0.3 | 0.0 |
| MOSS OAKMOSS SYNTHETIC | IMP | 0.0 | 0.0 | 0.0 | 1.8 |
| MOSS OAKMOSS SYNTHETIC 10% DPG | IMP | 0.0 | 0.2 | 0.0 | 0.0 |
| MUGUET MAYCIANE OIL | NA | 6.0 | 0.0 | 0.0 | 0.0 |
| MUSCENONE (TM) (F) | HMR | 0.0 | 0.0 | 0.4 | 0.0 |
| NONALACTONE GAMMA | NA | 0.0 | 0.0 | 0.1 | 0.0 |
| NONALACTONE GAMMA 10% DPG | NA | 0.7 | 0.0 | 0.0 | 0.0 |
| NUTMEG | IMP | 0.0 | 0.0 | 0.0 | 2.5 |
| OLIBANUM RESINOID | IMP | 0.0 | 0.0 | 0.0 | 1.0 |
| ORANGE BRAZIL PURE | HMI | 1.0 | 0.0 | 2.8 | 3.0 |
| ORTHOLATE (TM) (G) | HMI | 0.0 | 0.5 | 2.4 | 2.0 |
| OZONAL AB 7203C (TM) | IMP | 0.0 | 0.0 | 0.0 | 2.0 |
| PATCHOULI OIL | IMP | 0.0 | 0.0 | 0.0 | 5.0 |
| PHENYL ETHYL ALCOHOL | GEN | 3.3 | 0.8 | 0.0 | 0.0 |
| PRUNELLA (TM) (F) | RMP | 0.0 | 0.0 | 1.3 | 0.0 |
| RASPBERRY KETONE | HMP | 0.0 | 0.0 | 0.5 | 0.0 |
| ROSE OIL | NA | 0.0 | 0.3 | 0.0 | 0.0 |
| ROSE OXIDE LAEVO 10% DPG | IMP | 0.0 | 0.0 | 0.0 | 0.5 |
| ROSEMARY FRENCH OIL | IMP | 0.0 | 0.0 | 0.0 | 2.4 |
| SAGE CLARY OIL | IMP | 0.0 | 0.0 | 0.0 | 0.9 |
| SANDELA GD (TM) (G) | GEN | 0.0 | 0.5 | 1.8 | 2.0 |
| SILVANONE (TM) (G) | HMR | 0.0 | 2.0 | 0.0 | 0.0 |
| SPEARMINT OIL | IMP | 0.0 | 0.0 | 0.0 | 0.2 |
| STYRALLYL ACETATE | HMP | 0.3 | 0.0 | 0.3 | 0.0 |
| TARRAGON OIL | IMP | 0.0 | 0.0 | 0.0 | 0.4 |
| TERPINEOL | RMP | 1.2 | 0.0 | 0.0 | 0.0 |
| TUBEROSE BASE | NA | 0.6 | 0.0 | 0.0 | 0.0 |
| UNDECALACTONE GAMMA | HMR | 0.0 | 0.3 | 0.6 | 0.0 |
| UNDECAVERTOL (G) | HMP | 0.2 | 6.0 | 1.3 | 0.0 |
| VELOUTONE (F) | HMP | 0.0 | 0.0 | 0.3 | 0.0 |
| YLANG OIL | HMP | 0.9 | 0.0 | 0.0 | 0.0 |
| | total % | 100.0 | 100.0 | 100.0 | 100.0 |

| | | | | | |
|---|---|---|---|---|---|
| KEY B = BIOLANDES F = FIRMENICH G = GIVAUDAN IFF = INTERNATIONAL FLAVOR & FRAGRANCES Notes: 1) "Ingredient Y X% DPG" indicates X% of ingredient Y in a dipropylene glycol solution 2) "NA" indicates a material that is not allocated (i.e. the group is unknown). 3) "SOLV" indicates the material to be a solvent | | | | | |

**Table 5**

| GROUP ANALYSIS of Fragrance Compositions of Table 3 | | | | |
|---|---|---|---|---|
| **Group Sums %** | H2 | H3 | H4 | C4 |
| HMRs | 15.2 | 35.7 | 21.8 | 1.3 |
| HMIs | 6.0 | 8.9 | 8.0 | 15.7 |
| GENs | 45.6 | 37.0 | 41.6 | 9.6 |
| IMPs | 4.1 | 0.3 | 0.0 | 56.6 |
| HMPs | 4.6 | 10.1 | 9.7 | 0.2 |
| RMPs | 3.9 | 2.9 | 1.3 | 0.5 |
| TOTAL | 79.5 | 95.3 | 82.4 | 83.9 |
| SOLV | 11.8 | 3.4 | 16.1 | 11.5 |
| | | | | |

| **Solvent Corrected Sums** | H2 | H3 | H4 | C4 |
|---|---|---|---|---|
| HMRs | 17.3 | 37.0 | 26.0 | 1.5 |
| HMIs | 6.8 | 9.5 | 9.5 | 17.8 |
| GENs | 51.7 | 38.4 | 49.6 | 10.8 |
| IMPs | 4.7 | 0.3 | 0.0 | 63.9 |
| HMPs | 5.2 | 10.5 | 11.5 | 0.2 |
| RMPs | 4.4 | 3.0 | 1.6 | 0.6 |
| GENs + HMPs+HMIs+HMRs | 81.0 | 95.3 | 96.6 | 30.3 |
| TOTAL | 90.1 | 98.6 | 98.2 | 94.8 |
| | | | | |

| **Ratios** | H2 | H3 | H4 | C4 |
|---|---|---|---|---|
| HMPs/(HMPs+IMPs+RMPs) | 0.36 | 0.76 | 0.88 | 0.00 |
| HMPs/(HMPs+IMPs+RMPs+M) | 0.22 | 0.69 | 0.77 | 0.00 |

| | | | | |
|---|---|---|---|---|
| Key: TOTAL =HMPs+IMPs+RMPs+HMIs+HMRs+GENs M = 100-TOTAL | | | | |

## Claims

1. A method of providing a mood of well-being and happiness through the inhalation of an effective amount of a fragrance composition comprised of at least 75% by weight, preferably 85% by weight of perfume materials drawn from the following groups:
A) At least #L 1 0% by weight in total of at least three materials drawn from Group 'HMP' comprising: 1-(2,6,6,8-tetramethyltricyclo [5.3.1.0{1,5}]undec-8-en-9-yl)ethanone; allyl cyclohexylpropionate; allyl heptanoate; Apple Oliffac S pcmf; 7-methyl-2H-1,5-benzodioxepin-3(4H)-one; cassis base; cis-3-hexenyl salicylate; damascenone; gamma-decalactone; ethyl acetoacetate; ethyl maltol; ethyl methyl phenylglycidate; hexyl acetate; (3E)-4-methyldec-3-en-5-ol; 2,5,5-trimethyl-6,6-bis(methyloxy)hex-2-ene; 4-(4-hydroxyphenyl)butan-2-one; styrallyl acetate; 2,2,5-trimethyl-5-pentylcyclopentanone; ylang oil.
B) Optionally up to 95% of materials from the following groups:
Group 'HMR' comprising:
allyl ionone; benzyl acetate; cis-jasmone; citronellol; ethyl linalol; ethylene brassylate; 4-methyl-2-(2-methylpropyl)tetrahydro-2H-pyran-4-ol; geraniol;
geranium oil; iso-eugenol; lemon oil; 3-(4-hydroxy-4-methylpentyl) cyclohex-3-ene-1-carbaldehyde; 4-(4-hydroxy-4-methylpentyl)cyclohex-3-ene-1-carbaldehyde;
alpha-iso-methyl ionone; 3-methylcyclopentadec-2-en-1-one; cyclopentadecanone;
cyclohexadecanolide; gamma-undecalactone.
Group 'HMI' comprising:
1-{[2-(1,1-dimethylethyl)cyclohexyl]oxy}butan-2-ol; 3a,6,6,9a-tetramethyldodecahydronaphtho[2,1-{b}]furan; alpha-damascone;
dihydromyrcenol; eugenol; 3-(1,3-benzodioxol-5-yl)-2-methylpropanal;
2,4-dimethylcyclohex-3-ene-1-carbaldehyde; mandarin oil; orange oil;
2-(1,1-dimethylethyl)cyclohexyl acetate.
Group 'RMP' comprising:
anisic aldehyde; (2Z)-2-ethyl-4-(2,2,3-trimethylcyclopent-3-en-1-yl)but-2-en-1-ol;
benzoin siam resinoid; ethyl vanillin; oxacyclohexadec-12(13)-en-2-one; hexyl salicylate; hydroxycitronellal; jasmin oil; 3-methyl-5-phenylpentan-1-ol; 2-(phenyloxy)ethyl 2-methylpropanoate; alpha-terpineol; vanillin;
Group 'IMP' comprising:
allyl amyl glycolate; benzyl salicylate; bergamot oil; coriander oil; cyclamen aldehyde; 1-(2,6,10-trimethylcyclododeca-2,5,9-trien-1-yl)ethanone; prop-2-enyl (cyclohexyloxy)acetate; Damascenia 185 SAE; 2,4-dimethylheptan-1-ol; fir balsam;
fir needle oil; 3-(4-ethylphenyl)-2,2-dimethylpropanal; ginger oil; guaiacwood;
linalyl acetate; litsea cubeba oil; methyl 2,4-dihydroxy-3,6-dimethylbenzoate;
nutmeg oil; olibanum oil; orange flower oil; Ozonal AB 7203C; patchouli oil; rose oxide; rosemary oil; sage clary oil; spearmint oil; Tamarine AB 8212E; tarragon oil;
Group 'GEN' comprising:
cyclopentadecanolide; hexyl cinnamic aldehyde; ionone beta; isobornyl cyclohexanol; 1-(2,3,8,8-tetramethyl-1,2,3,4,5,6,7(8),8(8a)-octahydronaphthalen-2-yl)ethanone; 3-[4-(1,1-dimethylethyl)phenyl]-2-methylpropanal; linalol; methyl dihydrojasmonate; 2-phenylethanol; provided the following conditions are met:
(a) HMPs + HMRs >= IMPs
(b) HMPs + HMIs >= RMPs
(c) HMRs + HMIs + HMPs +GENs >= 65%
(d) HMPs/(HMPs+RMPs+IMPs) >= 0.35
(e) HMPs/[(HMPs+RMPs+IMPs) + (100-TOTAL)] >= 0.2
wherein 'HMPs' indicates the sum of the percentages of materials within Group HMP, and similarly for the remaining groups, the symbol '>=' indicates 'at least equal to', and 'TOTAL' is the sum of HMPs, HMRs, HMIs, IMPs, RMPs and GENs, provided also that low odour or no odour solvents are excluded from the calculation of these sums.

2. A method according to claim 1, which comprises at least five materials from Group HMP.

3. A method according to claim 2, which comprises at least eight materials from Group HMP.

4. A method according to any one preceding claim, which comprises at least one material from each of Groups HMR and HMI.

5. A method according to any one preceding claim, which comprises at least 5% by weight in total of materials drawn from the total of Groups HMR and HMI.

6. A method according to claim 5, wherein the total from Groups HMR and HMI is at least 10% by weight.

7. A method according to any one preceding claim, which comprises at least 75% by weight of materials selected from the Groups HMR, HMI, HMP and GEN.

8. A method according to any one preceding claim, wherein the ratio HMPs / (HMPs + RMPs + IMPs) is at least 0.55, preferably at least 0.75.

9. A method according to any one preceding claim which comprises at least 15 perfumery ingredients.

10. A method according to claim 9, which comprises at least 25 perfumery ingredients.

11. A method according to claim 10, which comprises at least 40 perfumery ingredients.

## Patentansprüche

1. Verfahren zum Vermitteln eines Gefühls von Wohlbefinden und Heiterkeit durch Einatmen einer wirksamen Menge einer Duftstoffzusammensetzung bestehend aus mindestens 75 Gew.-%, vorzugweise 85 Gew.-%, an Parfümmaterialien aus den folgenden Gruppen:
A) mindestens 10 Gew.-% insgesamt an mindestens drei Materialien aus der Gruppe "HMP", umfassend: 1- (2, 6, 6, 8-Tetramethyltricylo[5.3.1.0{1,5}]undec-8-en-9-yl)ethanon; Allylcyclohexylpropionat; Allylheptanoat; Apple Oliffac S pcmf; 7-Methyl-2H-1,5-benzodioxepin-3(4H)-on; Cassis-Base; cis-3-Hexenylsalicylat; Damascenon; gamma-Decalacton; Ethylacetoacetat; Ethylmaltol; Ethylmethylphenylglycidat; Hexylacetat; (3E)-4-Methyldec-3-en-5-ol, 2,5,5-Trimethyl-6,6-bis-(methyloxy)hex-2-en; 4-(4-Hydroxyphenyl)butan-2-on; Styrallylacetat; 2,2,5-Trimethyl-5-pentylcyclopentanon; Ylang-Öl.
B) gegebenenfalls bis zu 95% an Materialien aus den folgenden Gruppen:
Gruppe "HMR", umfassend:
Allylionon; Benzylacetat, cis-Jasmon; Citronellol; Ethyllinalol; Ethylenbrassylat; 4-Methyl-2-(2-methylpropyl)tetrahydro-2H-pyran-4-ol; Geraniol; Geranienöl; iso-Eugenol; Zitronenöl; 3-(4-Hydroxy-4-methylpentyl)cyclohex-3-en-1-carbaldehyd; 4-(4-Hydroxy-4-methylpentyl)cyclohex-3-en-1-carbaldehyd; alpha-iso-Methylionon; 3-Methylcyclopentadec-2-en-l-on; Cyclopentadecanon, Cyclohexadecanolid; gamma-Undecalacton.
Gruppe "HMI" umfassend:
1-{[2-(1,1-Dimethylethyl)cyclohexyl]oxy}butan-2-ol, 3a,6,6,9a-Tetramethyldodecahydronaphtho[2,1-{b}]furan; alpha-Damascon; Dihydromyrcenol; Eugenol; 3-(1,3-Benzodioxol-5-yl)-2-methylpropanal; 2,4-Dimethylcyclohex-3-en-1-carbaldehyd; Mandarinenöl, Orangenöl, 2-(1,1-Dimethylethyl)cyclohexylacetat.
Gruppe "RMP", umfassend:
Anisaldehyd; (22)-2-Ethyl-4-(2,2,3-trimethylcyclopent-3-en-1-yl)but-2-en-1-ol; Benzoinsiamresinoid; Ethylvanillin; Oxacyclohexadec-12(13)-en-2-on; Hexylsalicylat; Hydroxycitronellal; Jasminöl; 3-Methyl-5-phenylpentan-1-ol; 2-(Phenyloxy)ethyl-2-methylpropanoat; alpha-Terpineol; Vanillin.
Gruppe "IMP", umfassend:
Allylamylglycolat; Benzylsalicylat; Bergamotte-Öl; Korianderöl; Cyclamenaldehyd; 1-(2,6,10-Trimethylcyclododeca-2,5,9-trien-1-yl)ethanon; Prop-2-enyl(cyclohexyloxy)acetat; Damascenia 185 SAE; 2,4-Dimethylheptan-1-ol; Kanada-Balsam; Tannennadelöl; 3-(4-Ethylphenyl)-2,2-dimethylpropanal; Ingweröl; Guajakholz; Linalylacetat; Litseacubeba-Öl; Methyl-2,4-dihydroxy-3,6-dimethylbenzoat; Muskatnussöl; Olibanum-Öl; Orangenblütenöl; Ozonal AB 7203C; Patschuli-Öl; Rosenoxid; Rosmarinöl; Muskatellersalbeiöl; Krauseminzeöl; Tamarine AB 8212E; Estragonöl;
Gruppe "GEN", umfassend:
Cyclopentadecanolid; Hexylzimtsäurealdehyd; beta-Ionon; Isobornylcyclohexanol; 1-(2,3,8,8-Tetramethyl-1, 2, 3, 4, 5, 6, 7 (8) , 8 (8a)-octahydro-naphthalin-2-yl)ethanon; 3-[4-(1,1-Dimethylethyl)-phenyl]-2-methylpropanal; Linalol; Methyldihydrojasmonat; 2-Phenylethanol; mit der Maßgabe, dass den folgenden Bedingungen entsprochen wird:
(a) HMPs + HMRs >= IMPs
(b) HMPs + HMIs >= RMPs
(c) HMRs + HMIs + HMPs + GENs >= 65%
(d) HMPs/(HMPs+RMPs+IMPs) >= 0,35
(e) HMPs/[(HMPs+RMPs+IMPs)+(100-INSGESAMT)] >= 0,2
wobei "HMPs" die Summe der Prozentsätze an Materialien innerhalb der Gruppe HMP angibt, und analog dazu für die verbleibenden Gruppen das Symbol ">=" "mindestens gleich" bedeutet und
"INSGESAMT" die Summe aus HMPs, HMRs, HMIs, IMPs, RMPs und GENs bedeutet, sowie mit der Maßgabe, dass Lösungsmittel mit wenig oder keinem Geruch aus der Berechnung dieser Summen ausgeschlossen sind.

2. Verfahren nach Anspruch 1, das mindestens fünf Materialien aus der Gruppe HMP umfasst.

3. Verfahren nach Anspruch 2, das mindestens acht Materialien aus der Gruppe HMP umfasst.

4. Verfahren nach einem vorhergehenden Anspruch, das mindestens ein Material aus jeder der Gruppen HMR und HMI umfasst.

5. Verfahren nach einem vorhergehenden Anspruch, das mindestens 5 Gew.-% insgesamt an Materialien aus der Gesamtheit der Gruppen HMR und HMI umfasst.

6. Verfahren nach Anspruch 5, wobei die Gesamtheit der Gruppen HMR und HMI mindestens 10 Gew.-% beträgt.

7. Verfahren nach einem vorhergehenden Anspruch, das mindestens 75 Gew.-% an Materialien aus den Gruppen HMR, HMI, HMP und GEN umfasst.

8. Verfahren nach einem vorhergehenden Anspruch, wobei das Verhältnis HMPs/(HMPs+RMPs+IMPs) mindestens 0,55, vorzugsweise mindestens 0,75, beträgt.

9. Verfahren nach einem vorhergehenden Anspruch, das mindestens 15 Parfümeriebestandteile umfasst.

10. Verfahren nach Anspruch 9, das mindestens 25 Parfümeriebestandteile umfasst.

11. Verfahren nach Anspruch 10, das mindestens 40 Parfümeriebestandteile umfasst.

## Revendications

1. Procédé pour fournir un état de bien-être et du bonheur grâce à l'inhalation d'une quantité efficace d'une composition de parfum, composée d'au moins 75 % en poids, de préférence 85 % en poids de substances à base de parfum choisies parmi les groupes suivants :
A) Au moins 10 % en poids total d'au moins trois substances choisies parmi le Groupe 'HMP' comprenant : la 1-(2,6,6,8-tetraméthyltricyclo[5.3.1.0{1,5}]undéc-8-èn-9-yl)éthanone ; le cyclohexylpropionate d'allyle; l'heptanoate d'allyle; l'Apple Oliffac S pcmf ; la 7-méthyl-2H-1,5-benzodioxépin-3(4H)-one ; une base de cassis ; le salicylate de cis-3-hexényl ; la damascénone ; la gamma-décalactone ; l'acétoacétate d'éthyle ; l'éthylmaltol ; le méthylphénylglycidate d'éthyle ; l'acétate d'hexyle ; le (3E)-4-méthyldéc-én-5-ol ; le 2,5,5-triméthyl-6,6-bis(méthyloxy)hex-2-ène ; la 4-(4-hydroxyphényl)butan-2-one ; l'acétate de styrallyle ; la 2,2,5-triméthyl-5-pentylcyclopentanone ; l'essence d'ylang-ylang.
B) Eventuellement jusqu'à 95 % de substances parmi les groupes suivants :
Le groupe 'HMR' comprenant :
l'allylionone ; l'acétate de benzyle ; la cis-jasmone ; le citronellol ; l'éthyllinalol ; le brassylate d'éthylène ; le 4-méthyl-2-(2-méthylpropyl)tétrahydro-2H-pyran-4-ol ; le géraniol ; l'essence de géranium ; l'isoeugénol ; l'essence de citron ; le 3-(4-hydroxy-4-méthylpentyl)cyclohex-3-ène-1-carbaldéhyde ; le 4-(4-hydroxy-4-méthylpentyl)cyclohex-3-ène-1-carbaldéhyde ; l'alpha-isométhylionone ; la 3-méthylcyclopentadéc-2-én-1-one ; la cyclopentadécanone ; le cyclohexadécanolide ; la gamma-undécalactone.
Le groupe 'HMI' comprenant :
le 1-{[2-(1,1-diméthyléthyl)cyclohexyl]oxy}butan-2-ol ; le 3a,6,6,9a-tétraméthyldodécahydronaphto[2,1-{b}]furane ; l'alpha-damascone ; le dihydromyrcénol ; l'eugénol ; le 3-(1,3-benzodioxol-5-yl)-2-méthylpropanal ; le 2,4-diméthylcyclohex-3-éne-1-carbaldéhyde ; l'essence de mandarine ; l'essence d'orange ; l'acétate de 2-(1,1-diméthyléthyl)cyclohexyle.
Le groupe 'RMP' comprenant :
l'aldéhyde anisique ; le (2Z)-2-éthyl-4-(2,2,3-triméthylcyclopent-3-èn-1-yl)but-2-èn-1-ol ; le résinoïde de benjoin du Siam ; l'éthylvanilline ; l'oxacyclohexadéc-12(13)-én-2-one ; le salicylate d'hexyle ; l'hydroxycitronellal ; l'essence de jasmin ; le 3-méthyl-5-phénylpentan-1-ol ; le 2-méthylpropanoate de 2-(phényloxy)éthyle ; l'alpha-terpinéol ; la vanilline.
Le groupe 'IMP' comprenant :
l'amylglycolate d'allyle; le salicylate de benzyle ; l'essence de bergamote ; l'essence de coriandre ; l'aldéhyde cyclamen ; la 1-(2,6,10-triméthylcyclododéca-2,5,9-trién-1-yl)éthanone ; le (cyclohexyloxy)acétate de prop-2-ényle ; le Damascenia 185 SAE ; le 2,4-diméthylheptan-1-ol ; l'essence de sapin baumier ; l'essence d'aiguilles de pin ; le 3-(4-éthylphényl)-2,2-diméthylpropanal ; l'essence de gingembre ; le bois de gaïac ; l'acétate de linalyle ; l'essence de verveine exotique ; le 2,4-dihydroxy-3,6-diméthylbenzoate de méthyle ; l'essence de muscade ; l'essence d'oliban ; l'essence de fleur d'oranger ; l'Ozonal AB 7203 C ; de l'huile de patchouli ; l'oxyde de rose ; l'essence de romarin ; l'essence de sauge sclarée ; l'essence de menthe verte ; la Tamarine AB 8212E ; l'essence d'estragon.
Le groupe 'GEN' comprenant :
le cyclopentadécanolide ; l'aldéhyde hexylcinnamique ; la bêta-ionone ; l'isobornylcyclohexanol ; la 1-(2,3,8,8-tétraméthyl-1,2,3,4,5,6,7(8),8(8a)-octahydronaphtalén-2-yl)éthanone ; le 3-[4-(1,1-diméthyléthyl)phényl]-2-méthylpropanal ; le linalol ; le dihydrojasmonate de méthyle ; le 2-phényléthanol ; pour autant que les conditions suivantes soient réunies ;
(a) HMPs + HMRs >= IMPs
(b) HMPs + HMIs >= RMPs
(c) HMRs + HMIs + HMPs +GENs >= 65 %
(d) HMPs/(HMPs+RMPs+IMPs) >= 0,35
(e) HMPs/[(HMPs+RMPs+IMPs) + (100-TOTAL)] >= 0,2
'HMPs' signifiant la somme des pourcentages de substances dans le groupe HMP, et de façon similaire pour les groupes restants, le symbole '>=' signifiant 'supérieur ou égal à', et 'TOTAL' étant la somme de HMPs, HMRs, HMIs, IMPs, RMPs et GENs, à condition aussi que les solvants peu ou pas odorants soient exclus du calcul de ces sommes.

2. Procédé selon la revendication 1, qui comprend au moins cinq substances du groupe HMP.

3. Procédé selon la revendication 2, qui comprend au moins huit substances du groupe HMP.

4. Procédé selon l'une quelconque des revendications précédentes, qui comprend au moins une substance de chacun des groupes HMR et HMI.

5. Procédé selon l'une quelconque des revendications précédentes, qui comprend au moins 5 % en poids en total de substances choisies parmi le total des groupes HMR et HMI.

6. Procédé selon la revendication 5, le total des groupes HMR et HMI étant d'au moins 10 % en poids.

7. Procédé selon l'une quelconque des revendications précédentes, qui comprend au moins 75 % en poids de substances choisies parmi les groupes HMR, HMI, HMP et GEN.

8. Procédé selon l'une quelconque des revendications précédentes, le rapport HMPs/(HMPs + RMPs + IMPs) étant d'au moins 0,55, de préférence d'au moins 0,75.

9. Procédé selon l'une quelconque des revendications précédentes qui comprend au moins 15 ingrédients parfumants.

10. Procédé selon la revendication 9, qui comprend au moins 25 ingrédients parfumants.

11. Procédé selon la revendication 10, qui comprend au moins 40 ingrédients parfumants.
